## Europäisches Patentamt

## European Patent Office

## Office européen des brevets

(19)

(11) Veröffentlichungsnummer: **0 168 522**
A2

(12) # EUROPÄISCHE PATENTANMELDUNG

(21) Anmeldenummer: 84113180.8

(22) Anmeldetag: 02.11.84

(51) Int. Cl.⁴: **A 61 K 9/22**

(30) Priorität: 18.11.83 US 553228

(43) Veröffentlichungstag der Anmeldung: 22.01.86
**Patentblatt 86/4**

(84) Benannte Vertragsstaaten: **AT BE CH DE FR GB IT LI NL SE**

(71) Anmelder: **Akzo GmbH,
Postfach 10 01 49 Kasinostrasse 19-23,
D-5600 Wuppertal-1 (DE)**

(72) Erfinder: **Castro, Anthony, 710 Roscoe, Chicago Illinois 60657 (US)**

(54) **Wirkstoff enthaltende Formkörper.**

(57) Es werden Formkörper, insbesondere Tabletten u.dgl. beschrieben, die eine einheitliche Struktur aufweisen und die durch Trockenvermischen von 10 bis 90 Gew.-% pulverförmigen synthetischen thermoplastischen Polymermaterial und 10 bis 90 Gew.-% eines Wirkstoffs sowie Verpressen dieses Gemischs zum Formkörper erhalten werden. Bei den Wirkstoffen handelt es sich um Substanzen, die entweder auf Grund von Bildung von Agglomeraten oder Gelteilchen in Wasser schwer löslich oder schwer verteilbar sind oder die auf Grund ihrer chemischen Instabilität gegenüber Wasser in wäßriger Lösung nur kurze Zeit beständig sind. Als Wirkstoff wird vorzugsweise Ampicillin verwendet. Die Formkörper eignen sich besonders zur Abgabe derartiger Wirkstoffe, indem man den Wirkstoff enthaltenden Formkörper in ein Gefäß mit Zu- und Ablauf bringt und durch das Gefäß eine Flüssigkeit, vorzugsweise Wasser oder wäßrige Lösungen leitet, die den Wirkstoff über eine bestimmte Zeit hinweg in nahezu gleichmäßiger Konzentration mitnehmen. Auf diese Weise können auf gleichmäßige Weise Medikamente, Narkotika u.dgl. appliziert werden.

0168522
A3AM45128

## Wirkstoff enthaltende Formkörper

A k z o    GmbH

Wuppertal

Die Erfindung betrifft Wirkstoff enthaltende Formkörper, Verfahren zu deren Herstellung sowie deren Verwendung zur Freigabe einer im wesentlichen gleichmäßigen Wirkstoffmenge pro Zeiteinheit. Die Erfindung betrifft insbesondere Formkörper, deren Herstellung und Verwendung, die aktive Wirkstoffe wie Narkotika, pharmazeutische Präparate und Medikamente enthalten, bei deren Verabreichung es besonders gewünscht ist, die Freigabe des Wirkstoffs über eine bestimmte Zeit in einer nahezu konstanten Konzentration zu ermöglichen, insbesondere unter Zuhilfenahme eines fließenden Stroms einer Flüssigkeit, die als Transportmittel für den Wirkstoff dient.

Es hat sich als wünschenswert erwiesen, Wirkstoffe, insbesondere Narkotika, Medikamente und pharmazeutische Präparate in relativ konstanten Mengen innerhalb eines bestimmten Zeitraums freisetzen zu können. In einigen Anwendungsbereichen wurden bereits Vorrichtungen als Wirkstoffquelle zur gesteuerten Verabreichung bzw. Freigabe des Wirkstoffes angewandt. Bei diesen Vorrichtungen ging es letztlich darum, eine Möglichkeit zur Erreichung einer Wirkstoffreigabe nach einer Reaktion 0. Ordnung zu schaffen,

   A3AM45128

d.h., daß die pro Zeiteinheit freigesetzte Wirkstoffmenge zumindest während eines bestimmten Teils des Freigabezyklus konstant und von der Konzentration und Menge des in der Vorrichtung noch vorhandenen Wirkstoffes unabhängig ist. Eine echte Freigabe 0. Ordnung läßt sich jedoch mit den meisten bekannten Vorrichtungen normalerweise nicht über einen nennenswerten Zeitraum aufrechterhalten.

Ein weiteres Problem besteht in der Schaffung einer geeigneten Möglichkeit zur Verabreichung bestimmter Medikamente u.dgl. an Patienten. Hierbei handelt es sich um Medikamente, bei denen den Patienten eine volle medizinische Dosis nicht stoßweise z.B. durch Injektion in die Vene, den Muskel oder ein anderes Gewebe nur mit Schwierigkeiten verabreicht werden kann, da das auf derart abrupte Weise verabreichte Medikament für den Körper nicht so ohne weiteres verträglich ist. Somit muß die aus medizinischer Sicht geeignete Dosis über einen längeren Zeitraum z.B. über 10 bis 60 Minuten hinweg verabreicht werden.

Bei derartigen Medikamenten besteht häufig noch ein weiteres Problem, nämlich die Instabilität in wäßriger Lösung. So hat ein Arzneimittelhersteller in diesen Fällen nicht die Möglichkeit, ein Medikament in Form einer Lösung mit einer bestimmten Konzentration zur Verabreichung an den Patienten zur Verfügung zu stellen, da das Medikament bereits vor der Verabreichung auf Grund seiner geringen Stabilität in wäßriger Lösung abgebaut würde. In einigen Fällen kommt als weitere Komplikation hinzu, daß sich aus derartigen, vom Hersteller in trockener Form gelieferten Medikamenten die gewünschte wäßrige Lösung nur schwer herstellen läßt, da die Medikamente auf Grund der Bildung von Agglomeraten oder Gelteilchen in Wasser nur schwer löslich und/oder verteilbar sind.

Aufgabe der Erfindung ist es deshalb, aktive Wirkstoffe
enthaltende Formkörper, wie z.B. Tabletten zur Verfügung
zu stellen, welche Wirkstoffe der obengenannten Art enthalten, d.h. insbesondere solche, die in Wasser oder wäßrigen Lösungen Agglomerate oder Gelteilchen bilden oder auf
Grund ihrer chemischen Instabilität in wäßriger Lösung sich
dort verhältnismäßig schnell zersetzen.

Aufgabe der Erfindung ist ferner ein Verfahren zur Herstellung
derartiger Formkörper, insbesondere von tablettenförmigen
Formkörpern sowie deren Verwendung bei der kontrollierten
Abgabe von Wirkstoffen, wobei die Abgabe des Wirkstoffs über
einen bestimmten Zeitraum möglichst konstant bleiben soll.

Diese Aufgabe wird gelöst durch einen Formkörper mit einheitlicher Struktur aus ca. 10 bis 90 Gew.-% eines Wirkstoffes
und ca. 90 bis 10 Gew.-% eines synthetischen thermoplastischen
Polymermaterials, dadurch gekennzeichnet, daß der Formkörper
einen im wesentlichen physikalischen Zusammenhalt aufweist und
der Wirkstoff gleichmäßig in einer mit der Außenfläche des
Formkörpers kommunizierenden Weise im gesamten Formkörper
verteilt ist, wobei der Wirkstoff zwar eine Löslichkeit im
Wasser aufweist, er aber auf Grund der Bildung von Agglomeraten oder Gelteilchen in Wasser schwer löslich oder schwer verteilbar ist und/oder auf Grund seiner chemischen Instabilität
in wäßriger Lösung nur kurze Zeit beständig ist.

Es ist von Vorteil, wenn das Polymermaterial hydrophob ist.
Als Wirkstoff ist Ampicillin besonders geeignet.

Weitere sehr vorteilhafte Wirkstoffe sind:

Adrenokortikotropisches Hormin (ACTH), Bleomycin, Cytoxinarabinosid,Diphenylhydramin, ein gonadotropinfreisetzendes
Hormon (GnRH), Insulin, Parathormon, Angiotensin II, Chlordiacepoxyd, Diäthylcarbamizin, Dopamin, Heparin, Nitrofurantoin oder Pilocarpin.

Weitere sehr geeignete Wirkstoffe sind:

Mithramycin, Natrium-Warfarin, Pentothal, Daunorubicin-
Hydrochlorid, menschliches Choriongonadotropin, Amoxicillin,
Natrium-Cefazolin, Cephaloridin, Mitomycin, Cyclophosphamid,
Asparaginase, Natrium-Methicillin, Polymyxin-B-Sulfat,
Tetracyclin-Hydrochlorid, Minocyclin-Hydrochlorid, Kalium-
Penicillin V oder Natrium-Cloxacillin.

Es ist von Vorteil, wenn der Wirkstoff in Wasser eine Löslichkeit von mindestens 5 % aufweist.

Gegenstand der Erfindung ist ferner ein Verfahren zur Herstellung eines Formkörpers, dadurch gekennzeichnet, daß man
10 bis 90 Gew.-% Teilchen des Wirkstoffs mit 10 bis 90 Gew.-%
Polymerteilchen, wobei die Polymerteilchen einen Durchmesser
von ca. 40 bis ca. 400 µm aufweisen und zu ca. 0 bis ca.
100 Gew.-% aus mikroporösen Teilchen eines synthetischen thermoplastischen Polymermaterials und zu ca. 0 bis ca. 100 Gew.-%
aus einem nichtporösen synthetischen thermoplastischen Polymermaterial bestehen, trocken miteinander vermischt, sodann in
die gewünschte Form bringt und einem effektiven Druck von
ca. 200 bis 8000 psi aussetzt, wobei die Mischung in der gewünschten Form für einen Zeitraum gehalten wird, der ausreicht,
um der Mischung einen im wesentlichen physikalischen Zusammenhalt zu verleihen.

Besonders vorteilhaft ist, wenn man ein hydrophobes Polymermaterial verwendet. Das Verfahren läßt sich in besonders
vorteilhafter Weise auf Wirkstoffe anwenden, die in Wasser
eine Löslichkeit von mindestens 5 % aufweisen.

In einer besonders vorteilhaften Ausführungsform des
erfindungsgemäßen Verfahrens wird das Gemisch zu Tabletten
geformt.

Gegenstand der Erfindung ist ferner die Verwendung der vorstehend beschriebenen Formkörper zur Freisetzung einer im
wesentlichen gleichmäßigen Wirkstoffmenge pro Zeiteinheit,
dadurch gekennzeichnet, daß man ein Lösungsmittel für den
Wirkstoff mit gesteuerter Geschwindigkeit in ein mit einer
Einlaß- und einer Abflußöffnung, die in einem gewissen Abstand voneinander angebracht sind, versehenes Gefäß einleitet,
wobei das Gefäß so angeordnet ist, daß es darin zu keiner
wesentlichen Ansammlung von konzentrierter Wirkstofflösung
kommt, und wobei das Gefäß den Formkörper enthält und man
das Lösungsmittel, das den darin gelösten oder verteilten
Wirkstoff enthält, aus dem Gefäß durch die Abflußöffnung
mit einer gesteuerten Geschwindigkeit ableitet. Es ist vorteilhaft, wenn das Gefäß so angeordnet ist, daß die Ableitung
der Flüssigkeit aus dem Gefäß in Folge der Schwerkraft erfolgt.
Als tablettenförmiger Formkörper, der zur Freisetzung der
Wirkstoffmenge verwendet werden kann, ist ein tablettenförmiger Formkörper aus Polyamid und dem Wirkstoff
Ampicillin besonders geeignet, zweckmäßigerweise werden
dabei Formkörper aus gleichen Teilen Polyamid und Ampicillin
verwendet.

Kommunizierende Weise im Rahmen der Erfindung bedeutet, daß
die Hohlräume des Formkörpers, die aus Poren und Zellen be-

0168522

stehen, miteinander verbunden sind und es möglich ist, diese
Hohlräume mehr oder weniger vollständig mit einer Flüssigkeit zu füllen und auch wieder zu entleeren, wobei die
Flüssigkeit, wie bei kommunizierenden Röhren, vollständig
miteinander in Verbindung steht.

Nachstehend erfolgt eine kurze Erläuterung der Figuren, die
zum Verständnis der Erfindung dienen:

Fig. 1      ist eine schematische Darstellung der zum Nachweis
            der mit den in den Beispielen beschriebenen
            erfindungsgemäßen Strukturen erzielbaren Freisetzungsgeschwindigkeiten benutzten Apparatur.

Fig. 2      ist eine graphische Darstellung des gemäß Beispiel I
            der Erfindung freigesetzten Ampicillin-Anteils
            in % im Verhältnis zur Zeit.

Fig. 3      ist eine graphische Darstellung des gemäß Beispiel II
            der Erfindung freigesetzten Ampicillin-Anteils
            in % im Verhältnis zur Zeit.

Fig. 4      ist eine graphische Darstellung des gemäß Beispiel III,
            das zu Vergleichszwecken angeführt ist, freige-
            setzten Ampicillin-Anteils in % im Verhältnis zur
            Zeit.

Fig. 5      ist eine graphische Darstellung des gemäß Beispiel IV
            der Erfindung freigesetzten Ampicillin-Anteils
            in % im Verhältnis zur Zeit.

Fig. 6      ist eine graphische Darstellung des gemäß Beispiel V
            der Erfindung freigesetzten Ampicillin-Anteils
            in % im Verhältnis zur Zeit.

0168522

A3AM45128

Fig. 7,8
und 9        sind graphische Darstellungen des gemäß Beispiel
             VI-VIII freigesetzten Ampicillin-Anteils
             in % im Verhältnis zur Zeit.


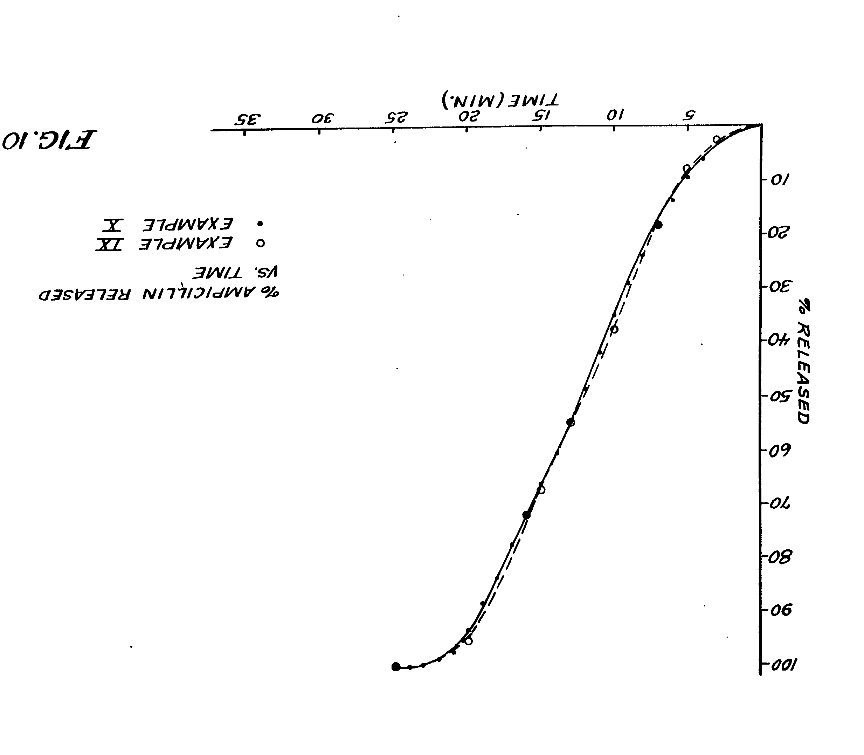

Fig. 10      zeigt graphische Darstellungen des gemäß Beispiel
             IX und X freigesetzten Ampicillin-Anteils
             in % im Verhältnis zur Zeit.


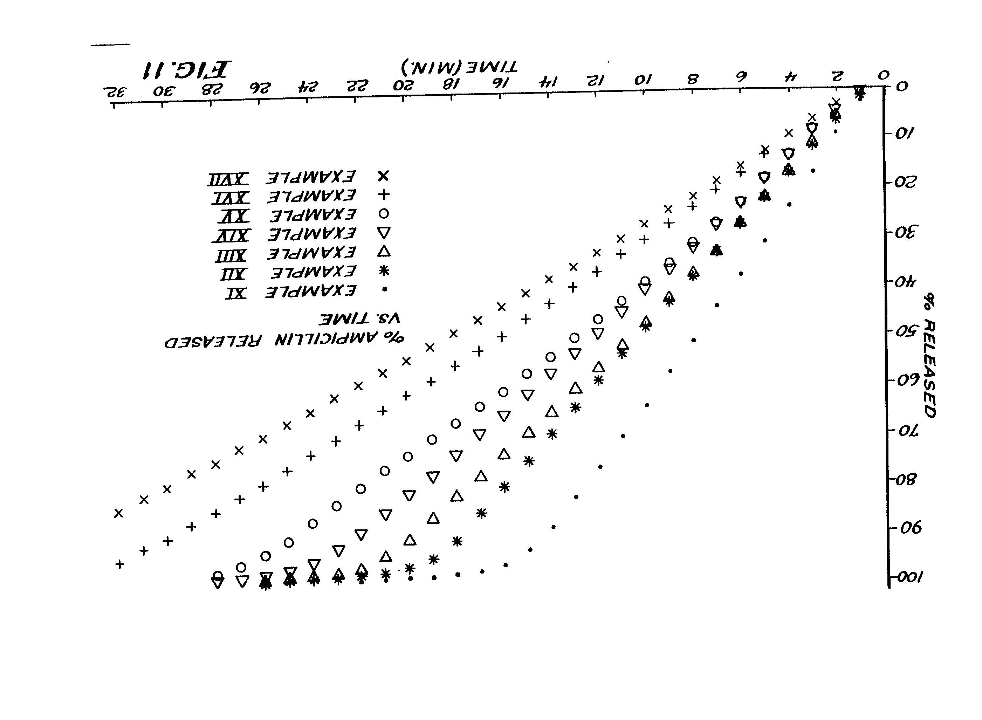

Fig. 11      zeigt graphische Darstellungen des gemäß Beispiel
             XI-XVII freigesetzten Ampicillin-Anteils
             in % im Verhältnis zur Zeit.


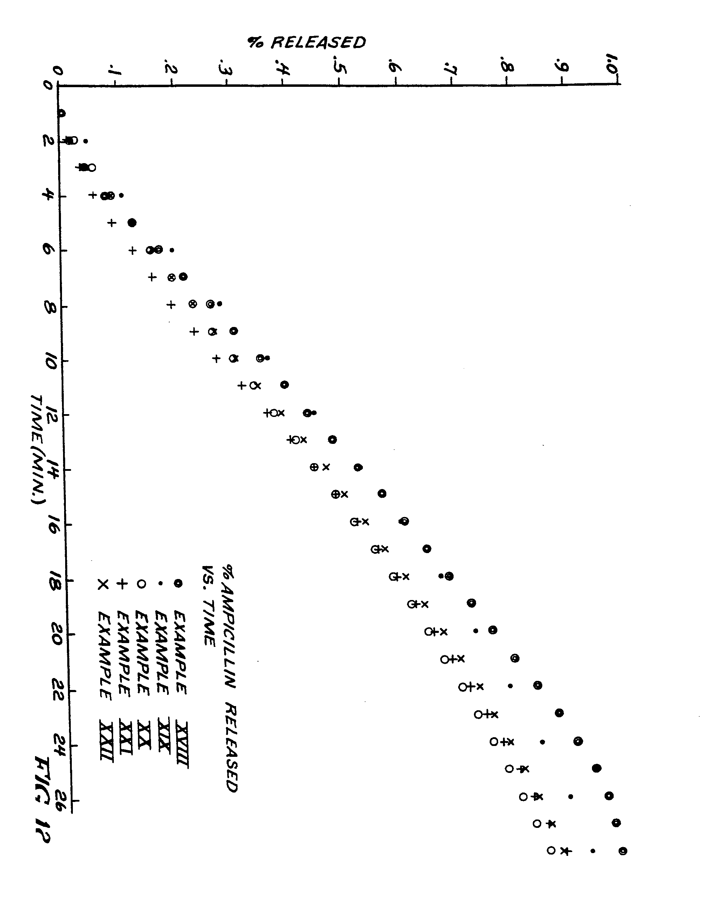

Fig. 12      zeigt graphische Darstellungen des gemäß Beispiel
             XVIII-XXII freigesetzten Ampicillin-Anteils
             in % im Verhältnis zur Zeit.


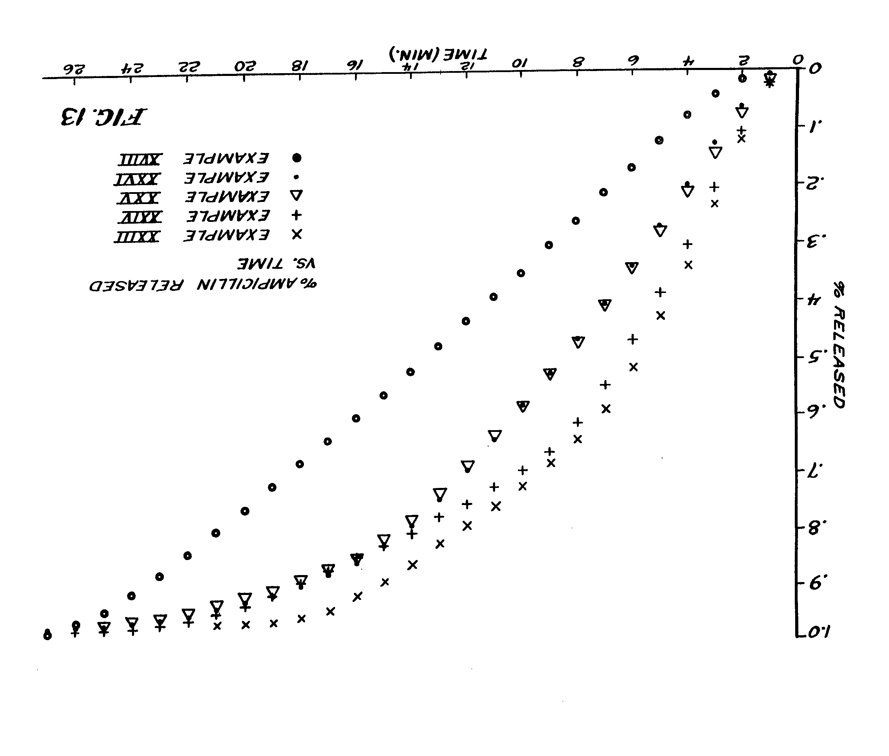

Fig. 13      zeigt graphische Darstellungen des gemäß Beispiel
             XXIII-XXVI freigesetzten Ampicillin-Anteils
             in % im Verhältnis zur Zeit im Vergleich zu
             Beispiel XVIII.


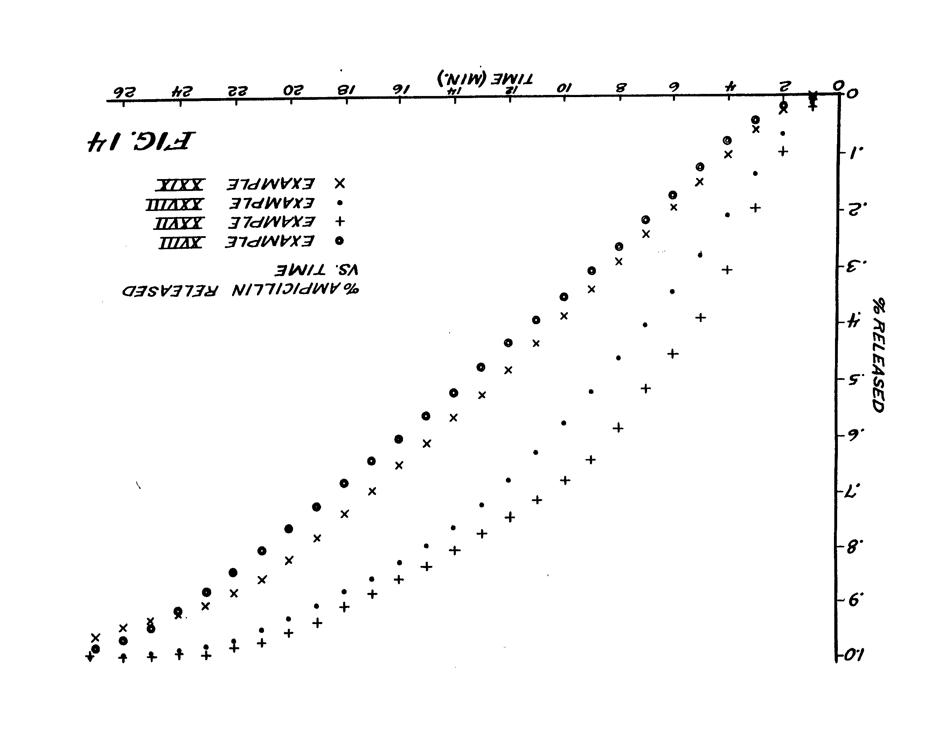

Fig. 14      zeigt graphische Darstellungen des gemäß Beispiel
             XXVII-XXIX freigesetzten Ampicillin-Anteils
             in % im Verhältnis zur Zeit im Vergleich zu
             Beispiel XVIII.


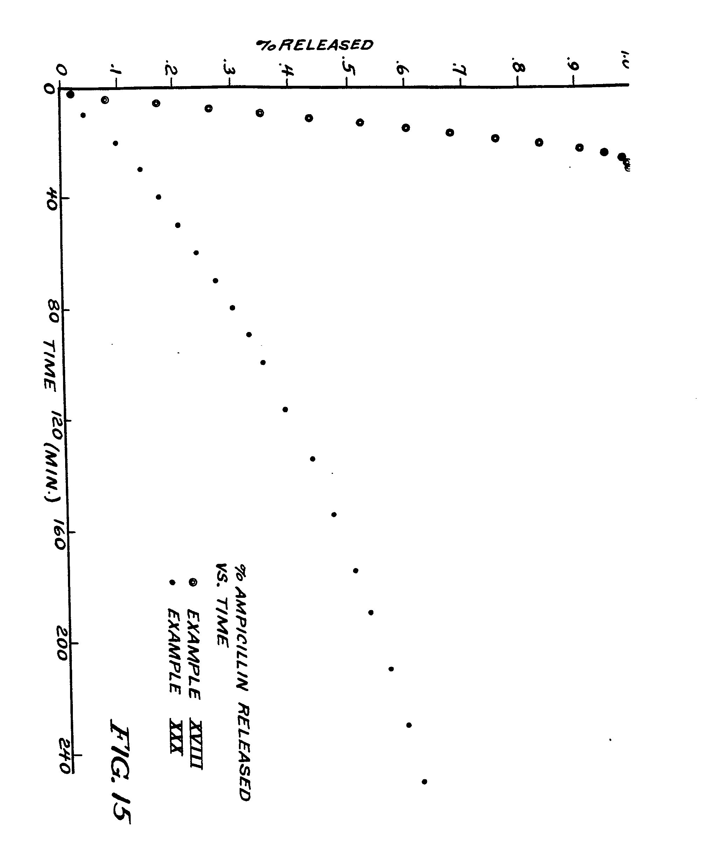

Fig. 15      zeigt graphische Darstellungen des gemäß Beispiel
             XXX freigesetzten Wirkstoffanteils (Acetaminophen)
             in % im Verhältnis zur Zeit im Vergleich zu
             Beispiel XVIII.


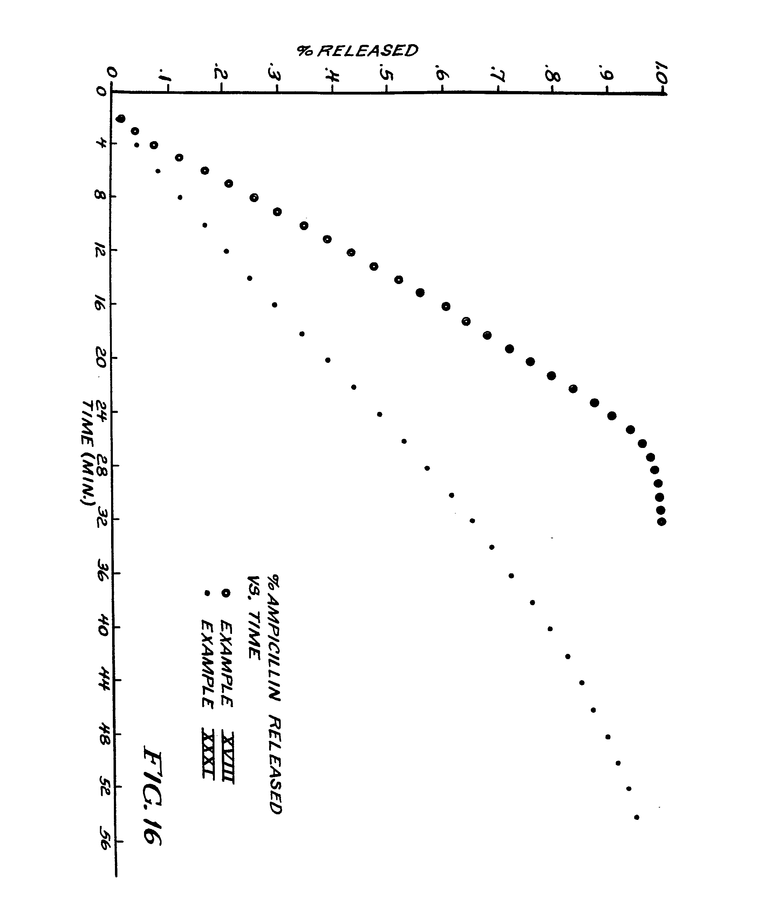

Fig. 16      zeigt graphische Darstellungen des gemäß Beispiel
             XXXI freigesetzten Wirkstoffanteils (Hydrochinon)
             in % im Verhältnis zur Zeit im Vergleich zu
             Beispiel XVIII.

A3AM45128

Im folgenden wird die Erfindung näher erläutert:

Wie bereits dargelegt, bezieht sich die vorliegende Erfindung auf eine aus einem Wirkstoff und einem inerten Material bestehende homogene Struktur, wobei der Begriff "homogen" im Sinne von "im wesentlichen gleichmäßig" bzw. einheitlicher Struktur verwendet wird.

Die Wirkstoffe, auf die sich die vorliegende Erfindung bezieht, befinden sich bei Raumtemperatur in festem Zustand. Einige der Wirkstoffe sind dadurch gekennzeichnet, daß sie schwerlöslich sind. Sie können also entweder eine niedrige Lösungsgeschwindigkeitskonstante aufweisen oder bei Kontakt mit dem Lösungsmittel Gelteilchen oder Agglomerate bilden. Einige Wirkstoffe können sich nach der Lösung im Lösungsmittel auch als instabil erweisen. Somit kann ein sich längere Zeit in gelöstem Zustand befindlicher Wirkstoff zu einer unwirksamen oder anderweitig unerwünschten Form abgebaut werden.

Die Wirkstoffe können eine beliebige Wirkung besitzen, d.h. es kann sich um Medikamente, Pestizide, Pharmazeutika, Narkotika o.dgl. handeln. Die vorliegende Erfindung ist insbesondere auf Wirkstoffe mit hoher Lösungsgeschwindigkeit in Wasser anwendbar. Typische Beispiele für die gemäß der vorliegenden Erfindung eingesetzten Wirkstoffe sind Pharmazeutika und Narkotika.

Die Wirkstoffe der vorliegenden Erfindung können als wäßrige Lösung oder als Lösung in anderen Lösungsmitteln angewandt werden. Wenn es sich bei dem Wirkstoff um ein Narkotikum oder ein anderes Pharmazeutikum handelt, ist das Lösungsmittel normalerweise ein pharmazeutisch unbedenkliches Lösungsmittel, z.B. Wasser. Oft werden zur Lösung von Narkotika oder Pharmazeutika auch Glukoselösungen verwendet. Weitere geeignete Lösungsmittel sind wäßrige Lösungen von Natriumchlorid,
Natriumlaktat, Dextrose und Natriumchlorid, Invertzucker
und laktierte Ringer-Lösung.

Die Wirkstoffe lassen sich in dem geeigneten Lösungsmittel,
normalerweise Wasser, vorzugsweise in einer Menge von
mindestens ca. 5 % lösen. Somit beträgt die Löslichkeit
der meisten Wirkstoffe in Wasser mindestens ca. 50 mg/ml
Wasser. Beträgt die Löslichkeit des Wirkstoffes in dem eingesetzten Lösungsmittel weniger als ca. 5 %, kommt es unter
gewissen Voraussetzungen nicht zu einer Wirkstoffreigabe in
der Größenordnung von Null.

Zu den medizinischen bzw. pharmazeutischen Wirkstoffen gehören die obigen Stoffe, die durch ihre selektive Wirkung
beim Patienten gekennzeichnet sind, die eher auf die Verabreichungsgeschwindigkeit als auf die effektive Dosis zurückzuführen ist. Sie sind nur beispielhaft erwähnt.

Bei der erstgenannten Gruppe von Stoffen, d.h. die Aufzählung
von adrenokortikotropischen Hormonen bis Pilocarpin ist die
selektive Wirkung eher auf die Verabreichungsgeschwindigkeit
als auf die Dosis zurückzuführen. Man ist also bei diesen
Stoffen besonders darauf angewiesen, sie innerhalb eines bestimmten Zeitraums kontrolliert abzugeben.

Die zweite Gruppe der Stoffe, d.h. die von Mithramycin bis
Natrium-Cloxacillin sind in wäßriger Lösung verhältnismäßig instabil, so daß von ihnen keine Lösungen auf Vorrat hergestellt
werden können.

Von besonderer Bedeutung ist der Wirkstoff Ampicillin, da er
in wäßriger Lösung instabil ist, sich wegen der Bildung von
Gelteilchen oder Agglomeraten schwer in Wasser lösen läßt und
dem Patienten innerhalb eines begrenzten Zeitraums verabreicht
werden muß.

In dem erfindungsgemäßen Verfahren zur Freigabe einer im
wesentlichen gleichmäßigen Wirkstoffmenge pro Zeiteinheit besteht die hierfür geeignete homogene Struktur aus
einem Wirkstoff und einem inerten Material in den angegebenen Mengen. Der inerte Bestandteil ist vorzugsweise ein
synthetisches thermoplastisches Polymermaterial. Man kann
jedoch davon ausgehen, daß jedes bei Raumtemperatur feste
und gegenüber dem Wirkstoff und dem in dem Verfahren eingesetzten Lösungsmittel inerte Material verwendet werden
kann. Somit gehören zu den Beispielen weiterer inerter
geeigneter Materialien auch Cellulose, Talkum u.dgl.

Für die Teilchen eines synthetischen thermoplastischen
Polymermaterials kommt jede geeignete Ausführung in Frage.
Die Teilchen bestehen normalerweise hauptsächlich aus einem
Polymer, können jedoch noch andere Bestandteile, z.B. Füllstoffe, Modifikatoren, Stabilisatoren u.dgl. enthalten. Im
übrigen kann es sich bei den Polymeren um Homopolymere,
Mischpolymerisate oder eine Mischung aus verschiedenen Homopolymeren und/oder Mischpolymerisaten handeln.

Das erfindungsgemäße Verfahren gilt für Olefinpolymere,
Kondensationspolymere und Oxydationspolymere.

Ein typisches synthetisches Polymermaterial ist ein Homopolymer aus Polypropylen oder Polyäthylen.

Beispiele für geeignete acrylfreie Polyolefine sind Niederdruck-Polyäthylen, Hochdruck-Polyäthylen, Polypropylen,
Polystyrol, Polyvinylchlorid, Acrylnitril-Butadien-Styrol-
Terpolymere, Styrol-Acrylnitril-Mischpolymerisat, Styrol-
Butadien-Mischpolymerisate, Poly(4-Methyl-Penten-1), Polybutylen, Polyvinylidenchlorid, Polyvinylbutyral, chloriertes
Polyäthylen, Äthylen-Vinylacetat-Mischpolymerisate, Polyvinylacetat und Polyvinylalkohol.

0168522

A3AM45128

Geeignete Acryl-Polyolefine sind Polymethyl-Methacrylat, Polymethyl-Acrylat, Äthylen-Acrylsäure-Mischpolymerisate und Äthylen-Acrylsäure-Metallsalz-Mischpolymerisate.

Ein repräsentatives Beispiel eines geeigneten Oxydations-polymers ist Polyphenylenoxyd. Geeignete Kondensations-polymere sind u.a. Polyäthylen-Terephthalat, Polybutylen-Terephthalat, Polyamid 6, Polyamid 11, Polyamid 13, Polyamid 66, Polykarbonate und Polysulphone.

Die in einer Ausführung der vorliegenden Erfindung anwendbaren nicht-porösen Teilchen erhält man durch einfaches Mahlen und Sieben von festem Harz einer beliebigen körperlichen Form. Derartige Teilchen sind auch im Handel erhältlich.

Die mikroporösen Teilchen lassen sich zwar mit allen Verfahren herstellen, die derartige Teilchen aus dem gewünschten synthetischen Polymermaterial ergeben; bevorzugt eingesetzt wird jedoch das Verfahren gemäß U.S. Patent Nr. 4,247,498. Mit diesem Verfahren kann man zunächst eine Ausgangsstruktur einer beliebigen Form herstellen, vorzugsweise in einer leicht herstellbaren und mahlbaren Form. Diese Ausgangsstruktur kann dann mit Hilfe von im Handel für diesen Zweck erhältlichen Vorrichtungen gefriergemahlen und danach extrahiert werden. Die sich dabei ergebenden Teilchen lassen sich dann nach Teilchengrößen sortieren. Bevorzugt gelangen Teilchen in der Größenordnung von ca. 40 - ca. 400 µm zum Einsatz.

Bei der vorliegenden Erfindung sollten vzw. mindestens 5 % der eingesetzten Teilchen mikroporös sein und ein hohes Porenvolumen, z.B. ca. 50 - ca. 90 %, vorzugsweise ca. 70 - ca. 80 %, besitzen. Die Größe der Mikroporen ist nicht kritisch und liegt normalerweise im Bereich von ca. 0.05 µm

bis ca. 5 μm, wobei die typischeren Werte bei ca. 0,2 -
ca. 1,0 μm liegen.

Der Wirkstoff und die Teilchen des inerten Materials werden
im Trockenzustand auf geeignete Weise, z.B. durch einfaches
Mischen, miteinander verbunden, um eine gleichmäßige Verteilung des Wirkstoffes und der Teilchen des inerten
Materials zu erhalten. Danach wird die Mischung in die gewünschte Form gebracht.

Da bei der praktischen Durchführung der vorliegenden Erfindung
verwendete inerte Material ist vorzugsweise hydrophob. Hierbei bedeutet der Begriff "hydrophob" als Beschreibung eines
inerten Materials, daß das inerte Material eine Oberflächenspannung unter ca. 48 Dyn/cm besitzt. Analog dazu bezieht
sich der Begriff "hydrophil" auf ein Material mit einer Oberflächenspannung über ca. 48 Dyn/cm. Bei Einsatz eines nicht
hydrophoben inerten Materials in der vorliegenden Erfindung
kann es sich als erforderlich erweisen, das Verhältnis zwischen
dem Wirkstoff und dem inerten Material herabzusetzen, um
das für ein bestimmtes System gewünschte Freisetzungsverhalten
zu erreichen.

Der Wirkstoffgehalt der Mischung kann sehr unterschiedlich
sein. Eine Untergrenze ist nur durch die Praxis gegeben. Vorrichtungen mit einer Wirkstoffmenge unter ca. 10 % dürften
sich als wirtschaftlich unzweckmäßig erweisen, wobei es jedoch einige Anwendungen geben kann, bei denen Vorrichtungen
mit einem so niedrigen Wirkstoffgehalt zum Einsatz gelangen.
Somit beträgt der typische Wirkstoffgehalt der erfindungsgemäßen Vorrichtungen mindestens ca. 10 Gew.-%. Nach oben wird
die Wirkstoffmenge normalerweise nur durch die physikalische
Festigkeit der Vorrichtung begrenzt. Somit ist mit ca.
90 Gew.-% Wirkstoff fast die praktische Obergrenze erreicht,

 A3AM45128

wenn man eine Vorrichtung mit einer physikalischen Festigkeit, die eine Handhabung ohne einen wesentlichen physikalischen Abbau gestattet, erhalten will. Bei Verwendung
eines hydrophilen inerten Materials läßt sich die maximale
Wirkstoffmenge, mit der sich der gewünschte Erfolg erzielen
läßt, sogar auf 50 Gew.-% der Struktur oder weniger reduzieren. Unter diesen Umständen kann der Einsatz einer zu
großen Wirkstoffmenge unter bestimmten Umweltbedingungen
zu einer nicht im Null-Bereich liegenden Freigabe führen.

Wie bereits erwähnt, kommt für die vorliegende Erfindung
praktisch jede beliebige Form in Frage. Insbesondere können
Tabletten oder Scheiben geformt werden, wobei einfache
Vorrichtungen verwendet werden, um die Teilchen in der
gewünschten Form zu halten. Auf die Teilchen.
wird von außen Druck angewandt, z.B. unter Einsatz einer
hydraulischen Presse, wie sie zur Herstellung von KB-
Tabletten für die Infrarotspektroskopie verwendet wird.

Der angewandte Druck kann mindestens ca. 200 psi betragen.
Normalerweise erhält man eine beträchtliche Festigkeit,
wenn der angewandte Druck ca. 300 psi oder mehr, z.B. bis
zu ca. 14.000 psi, beträgt. Bei der Verwendung von mikroporösen Polymerteilchen ist es möglich, den Druck zu erniedrigen, der notwendig ist, um einen ausreichenden Zusammenhalt
und eine ausreichende Festigkeit des erhaltenen Formkörpers
zu erzielen.

Die Strukturen der vorliegenden Erfindung sind dadurch gekennzeichnet, daß der Wirkstoff in einer mit der Außenfläche
der Struktur flüssig kommunizierenden Weise in der gesamten
Struktur verteilt ist. So kann das Lösungsmittel nach längerem
Kontakt mit der Vorrichtung mit dem Wirkstoff in Berührung
kommen, wobei die Lösung des Wirkstoffs an oder in der Nähe der Außenfläche der Struktur beginnt und sich in die gesamte Struktur fortsetzt. Ein wichtiger Aspekt der vorliegenden Strukturen ist, daß der Kontakt eines Lösungsmittels mit dem Wirkstoff nicht auf die Diffusion beschränkt ist, wie dies bei zahlreichen dem Stand der Technik entsprechenden Vorrichtungen der Fall ist. So ist es nicht erforderlich, daß das Lösungsmittel durch das in den erfindungsgemäßen Strukturen vorhandene Polymermaterial diffundiert, um an den Wirkstoff zu gelangen und diesen zu lösen. und dann wieder durch eine Polymerbarriere aus der Struktur als Lösung durch Diffusion auszutreten. Im Gegenteil: In den vorliegenden Strukturen ist der Wirkstoff für das Lösungsmittel leicht zugänglich, ohne daß dieser Zugang auf die Diffusion beschränkt wäre. Man geht davon aus, daß die Bewegung der Wirkstofflösung durch eine Verbindung von Diffusion, Konvektion und/oder mechanischer Wirkung zustande kommt und somit wesentlich schneller ist, als zu erwarten wäre, wenn die Steuerung der Geschwindigkeit allein durch die Diffusion durch das Lösungsmittel bewirkt würde.

Der Wirkstoff in den Strukturen ist durch eine Reihe von miteinander verbundenen Gängen oder Kanälen von bestimmter Größe verbunden. So nimmt man an, daß sich der Wirkstoff in beträchtlichen Mengen in Kanälen oder Gängen befindet, die in der Größenordnung von ca. 20 bis ca. 100 µm liegen.

Wie bereits erwähnt, bezieht sich die vorliegende Erfindung auch auf ein Verfahren zur Freisetzung einer im wesentlichen gleichmäßigen Wirkstoffmenge pro Zeiteinheit. So wird im Normalfall während einer gewünschten Zeitspanne, z.B. der zur Freigabe von ca. 10 bis ca. 90 % der wünschten Wirkstoffmenge erforderlichen Zeit, die pro Zeiteinheit freigesetzte Wirkstoffmenge, d.h. die Freisetzungsgeschwindigkeit, höchstens um ca. + 10 %, vorzugsweise jedoch nur $\pm$ 5 % schwanken.

Bei dem vorgenannten Verfahren wird ein Lösungsmittel für den Wirkstoff mit einer gesteuerten Geschwindigkeit in ein geeignetes Gefäß durch dessen Einlaßöffnung geleitet. Die gesteuerte Geschwindigkeit kann innerhalb eines beliebigen Bereichs liegen. Typische Beispiele sind ca. 0,5 - ca. 3.0 ml/min, vorzugsweise ca. 1,7 ml.

Als Gefäß eignet sich jeder Behälter, der eine Einlaß- und eine Abflußöffnung besitzt, die in einem gewissen Abstand voneinander angeordnet sind, und so konstruiert ist, daß das mit dem Lösungsmittel und dem Wirkstoff in Berührung kommende Material nicht mit dem Lösungsmittel oder dem Wirkstoff chemisch reagiert.

Das Gefäß muß so positioniert sein, daß es innerhalb des Gefäßes zu keiner wesentlichen Ansammlung von konzentrierter Wirkstofflösung kommt. Nimmt das Gefäß z.B. eine Position ein, bei der sich die Abflußöffnung über der Einlaßöffnung befindet, so wird die entstandene Lösung, die schwerer ist als Wasser, zur Einlaßöffnung hin absinken, wodurch es zu einer wesentlichen Ansammlung von konzentrierter Wirkstofflösung und einem nicht wünschenswerten Freisetzungsverhalten kommt.

Vorzugsweise sollten die Einlaß- und die Abflußöffnung so angeordnet sein, daß sich die Abflußöffnung während der Durchführung des Verfahrens unterhalb der Einlaßöffnung befindet. Somit wird das Gefäß so angeordnet, daß die darin enthaltene Flüssigkeit, sofern keine andere Kraft als die Schwerkraft darauf einwirkt, vorzugsweise durch die Abflußöffnung austreten, auch wenn der Abfluß der Flüssigkeit aus dem Gefäß so verläuft, daß unter den gegebenen Umständen eine gewisse Menge Flüssigkeit sowohl durch die Einlaß- als auch durch die Abflußöffnung aus dem Gefäß austritt.

Bei dem genannten Verfahren enthält das Gefäß die oben beschriebenen erfindungsgemäßen Formkörper. Bei einer
bevorzugten Ausführung liegt die den Wirkstoff enthaltende
Struktur in Form einer Scheibe mit einem Durchmesser und
einem Querschnitt, die im wesentlichen dem Innendurchmesser
bzw. -querschnitt des Gefäßes entsprechen, vor. Vorzugsweise
liegen die Einlaß- und die Abflußöffnung an entgegengesetzten
Seiten des Gefäßes, und der den Wirkstoff enthaltende Formkörper liegt zwischen der Einlaß- und der Abflußöffnung.
Es werden auch andere Ausführungen in Erwägung gezogen, bei
denen sich der den Wirkstoff enthaltende Körper an einer
anderen Stelle innerhalb des Gefäßes befindet.

Das den gelösten Wirkstoff enthaltende Lösungsmittel wird
aus dem Gefäß durch die Abflußöffnung im wesentlichen mit
der gleichen gesteuerten Geschwindigkeit entfernt, mit der
das Lösungsmittel durch die Einlaßöffnung in das Gefäß eingeleitet wird. Ist das Gefäß noch nicht mit Flüssigkeit gefüllt,
wird natürlich zunächst eine gewisse Einfüllzeit benötigt,
während der weniger Flüssigkeit durch die Abflußöffnung austritt als durch die Einlaßöffnung zugeführt wird. Vorzugsweise wartet man zunächst, bis sich das Gefäß mit Flüssigkeit
gefüllt hat, wozu man, z.B. die Einlaß- und die Abflußöffnung
umdreht, um soviel Luft aus der Abflußöffnung herauszudrücken,
bis das Gefäß mit Flüssigkeit gefüllt ist, und dann das Gefäß
wieder in die geeignete Lage bringt, bei der sich die Einlaßöffnung oberhalb der Abflußöffnung befindet. Für den
Fachmann ist klar, daß das erste Einfüllen auch auf eine andere
geeignete Weise erfolgen kann, z.B. dadurch, daß man in den
Ableitungsbereich Ventile und in den obersten Teil des Gefäßes Luftventile einbaut.

Die folgenden Beispiele dienen der weiteren Veranschaulichung
der Erfindung, ohne diese jedoch darauf zu beschränken.

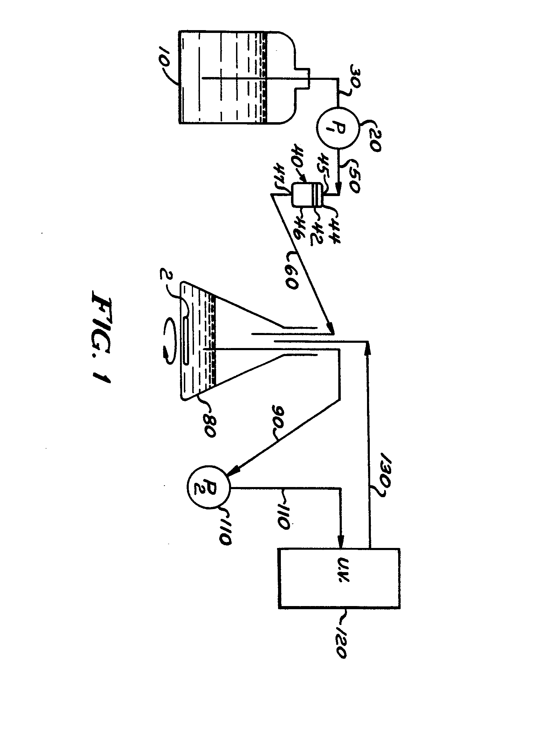

Abb. 1 ist eine schematische Darstellung der zum Nachweis der mit den erfindungsgemäßen Strukturen in den folgenden Beispielen erzielbaren Freisetzungsgeschwindigkeiten verwendeten Apparatur. Eine Vorrichtung zur Verabreichung eines Wirkstoffes, z.B. an einen Patienten, würde eines oder mehrere, jedoch nicht unbedingt alle der gezeigten Elemente enthalten, was von den unter den gegebenen Praxisbedingungen gewünschtem Ergebnis abhängt. In Abb. 1 zieht die Pumpe (20) durch die Leitung (30) aus dem Behälter (10) Flüssigkeit ab und leitet diese durch die Leitung (50) in das Gefäß (40) ein. Gefäß (40) besteht aus dem rostfreien Stahl (316), gliedert sich durch die Wand (42) in zwei Teile, (44) und (46), und hat eine Innenlänge von 1,26 in. und einen runden Innenquerschnitt mit einem Durchmesser von 1,5 in. Gefäß (40) besitzt ebenfalls eine Einlaßöffnung (45) und eine Abflußöffnung (47). Die Leitung (50) ist mit der Einlaßöffnung (45), und die Abflußöffnung (47) ist mit der Leitung (60) verbunden, die die Flüssigkeit von Gefäß (40) in Kammer (80), bei der es sich um einen Becher oder einen Kolben mit einem Fassungsvermögen von 1000 ml handelt, einleitet. Eine Möglichkeit zum kontinuierlichen Rühren der Flüssigkeit in Kammer (80) ist ebenfalls vorgesehen, z.B. in Form des Rührstabes (2). Die Leitung (90) ist mit der Pumpe (100) verbunden, die Flüssigkeit aus der Kammer (80) abzieht und durch die Leitung (110) dem UV-Spektrophotometer (120), z.B. Model Nr. 100-80 von Hitachi, zuführt. Die Leitung (130) ist mit der Abflußöffnung des Spektrophotometers verbunden und leitet die Flüssigkeit zurück in Kammer (80).

Wenn sich die Apparatur in Betrieb befindet, ist das Gefäß (40) mit einer einen Wirkstoff enthaltenden erfindungsgemäßen Struktur versehen, und in dieses Gefäß läßt man zunächst Flüssigkeit, z.B. Wasser, aus dem Behälter (10) einlaufen.
Die Pumpe (20) wird so eingestellt, daß die Flüssigkeit
mit einer bestimmten Geschwindigkeit vom Behälter (10)
zum Gefäß (40) und von dort in die Kammer (80) transportiert wird. Entsprechend wird auch die Pumpe (100) so eingestellt, daß die Flüssigkeit mit einer bestimmten Geschwindigkeit von der Kammer (80) zum Spektrophotometer
(120) fließt. Kammer (80) wird zunächst mit einer großen
Wassermenge, z.B. 1000 ml, gefüllt. Während also Flüssigkeit
von Behälter (10) in Gefäß (40) strömt und den Wirkstoff
löst, fließt wirkstoffhaltige Lösung von Gefäß (40) in
Kammer (80). Mit dem Spektrophotometer (120) wird die
Wirkstoffkonzentration in Gefäß (80) laufend erfaßt und
somit die Gesamtmenge des Gefäßes (80) im Verhältnis zur
Zeit zugeführten Wirkstoffs gemessen.

Es war besonders überraschend, daß es mit der Erfindung möglich ist, Wirkstoffe in kontrollierter Weise abzugeben. Insbesondere auch von Stoffen, die in Wasser oder wäßrigen
Lösungen nur wenig löslich sind bzw. schwer verteilbar oder
dispergierbar sind.

Die Erfindung wird durch folgende Beispiele näher erläutert:

Beispiel I

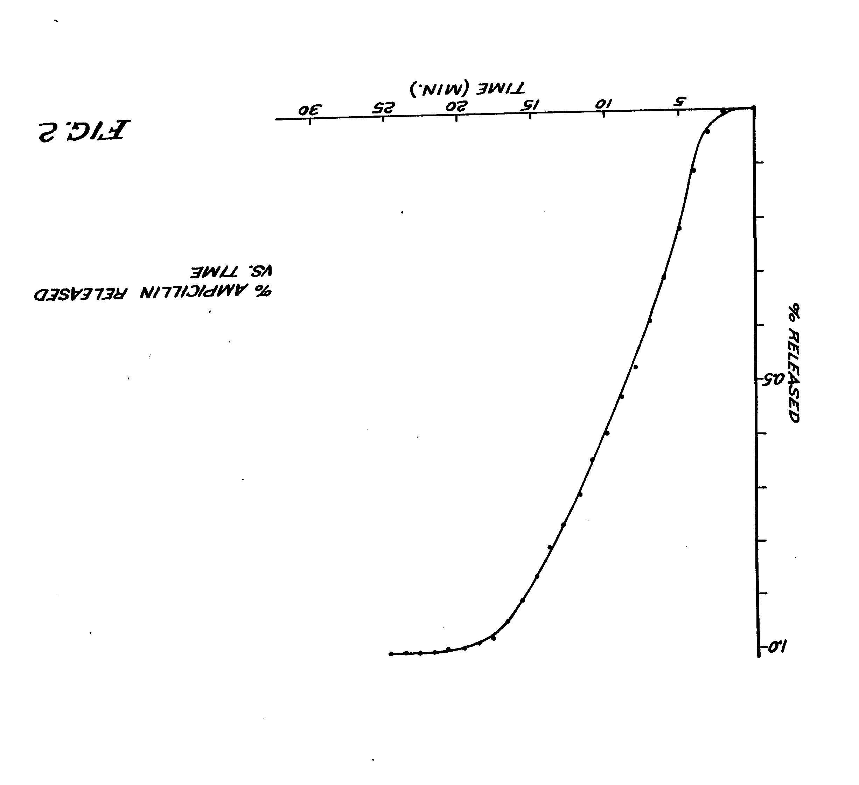

1 g Ampicillin wurde mit 0,17 g eines von der Fa. Hercules
unter dem Warenzeichen Pro-Fax 6323PM bezogenen nichtporösen Polypropylenpulvers,bei dem 80 % der Teilchen einen
Durchmesser zwischen 180 und 280 μm aufwiesen, gemischt.
Die Mischung wurde in einen Zylinder gegeben und in einer
hydraulischen Presse zu Tabletten mit einem Durchmesser von
3,175 cm bei einem Druck von 8000 psi gepreßt. Die Tablette
wurde dann in Gefäß (40) (s. Fig. 1) gegeben und die Pumpe (20)
auf eine Durchflußgeschwindigkeit von 100 ml/h eingestellt,
nachdem zunächst das Gefäß durch Umkehren mit einer Flüssigkeit gefüllt worden war. Der Vorratsbehälter (10) enthielt
eine 5%ige wäßrige Glucoselösung. Abb. 2 ist eine graphische
Darstellung des im Verhältnis zur Zeit freigesetzten Ampicillins
in %, aus der hervorgeht, daß die Gesamtmenge innerhalb eines
Zeitraums von ca. 20 Minuten freigesetzt wurde, wobei die Freisetzungsgeschwindigkeit in einem Zeitraum von ca. 3 - ca. 18
Minuten, auf den die Freisetzung von ca. 5 bis 95 % der gesamten vorhandenen Ampicillinmenge entfiel, nahezu konstant blieb.

Beispiel II

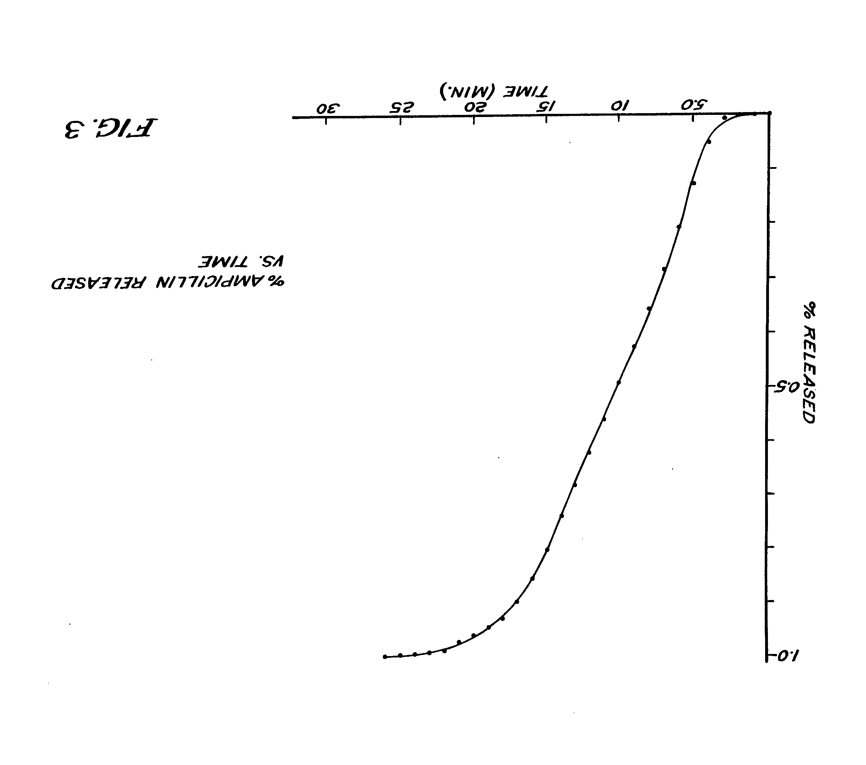

Analog zu Beispiel I wurde aus 1,0 g Ampicillin und 0,17 g
nichtporösem Polypropylen jedoch unter Anwendung eines Preßdruckes von 3000 psi eine Tablette hergestellt. Die Tablette
wurde wie in Beispiel I mit einer 5%igen Glucoselösung mit
einer Geschwindigkeit von 100 Ø ml/h getestet. Abb. 3 zeigt
eine graphische Darstellung des im Verhältnis zur Zeit freigesetzten Ampicillins in %, aus der hervorgeht, daß der gesamte
Freisetzungsvorgang ca. 24 Minuten in Anspruch nahm, wobei
die Freisetzungsgeschwindigkeit in einem Zeitraum von ca.
4 - ca. 18 Minuten, auf den die Freisetzung von ca. 5 bis ca. 90 Prozent der gesamten vorhandenen Ampicillinmenge
entfiel, nahezu konstant blieb.

Obwohl für eine vollständige Leerung der Struktur von
Beispiel I und II unterschiedliche Zeiten erforderlich
sind, ist die tatsächliche Zeit bei einer vergleichbaren
prozentualen Leerung bei beiden Beispielen im wesentlichen
gleich, wobei der Hauptgrund für den Unterschied in dem
abweichenden Beginn der zeitlichen Verzögerung liegt, wie
aus Abb. 2 und 3 hervorgeht.

## Beispiel III - Vergleichsbeispiel

Dieses Beispiel wurde durchgeführt um nachzuweisen, daß die
Anordnungsweise der Einlaß- und der Abflußöffnung von Gefäß
(40) sich auf das Freisetzungsverhalten des Systems auswirkt.
Zunächst wurde eine Tablette, wie in Beispiel I, jedoch
unter Verwendung von 0,86 g Ampicillin und 0,14 g mikroporösen Polypropylens, das als mikroporöses Pulver von der
Armak Company unter dem Warenzeichen Accurel[R] erhältlich
ist, hergestellt. Das Pulver wird nach dem Verfahren gemäß
US Patent 4,247,498 aus 25 Gew.-% Polypropylen und 75 Gew.-%
Armostat[R] 310 N,N-bis-(2-Hydroxyäthyl)-Talkamin) hergestellt. Die sich daraus ergebende Struktur wurde zu mikroporösen
Teilchen gefriervermahlen und dann zwecks Entfernung des Amins
mit Isopropanol extrahiert, getrocknet und zur Entfernung der
Feinteilchen gesiebt, so daß die dabei erhaltenen Teilchen
sich in der Größenordnung von ca. 40 bis ca. 400 µm bewegten.
Nach dem Mischen des Ampicillins mit dem Pulver wurde bei
einem Druck von 3000 psi eine Tablette geformt.

Die Tablette wurde in der gleichen Weise wie in Beispiel I
verarbeitet, wobei jedoch das Gefäß (40) umgekehrt wurde, so daß die Einlaßöffnung unterhalb der Ablußöffnung lag.

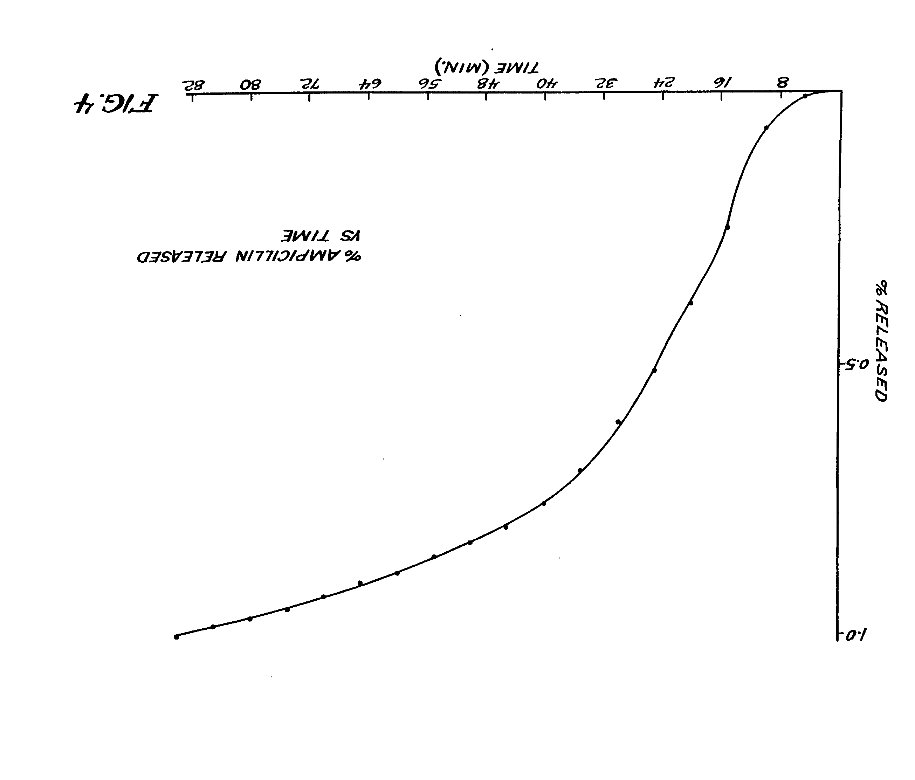

Abb. 4 ist eine graphische Darstellung des im Verhältnis
zur Zeit freigesetzten Ampicillins in %, aus der hervorgeht, daß der gesamte Freisetzungsvorgang ca. 88 Minuten
dauerte, wobei als Flüssigkeit in dem Bad mit konstanter
Temperatur Wasser eingesetzt wurde und die Fließgeschwindigkeit 100 ml/h betrug. Eine konstante Freisetzung über
einen nennenswerten Teil der Freisetzungskurve war nicht
festzustellen.

Beispiel IV

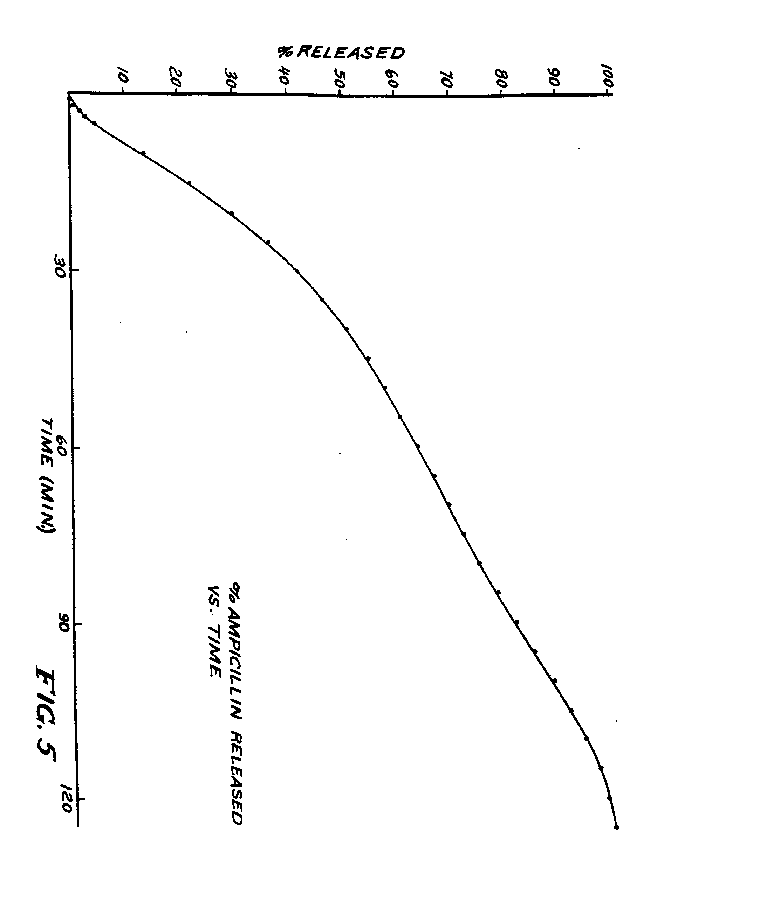

Gemäß dem Verfahren von Beispiel I wurde aus 1,88 g
Ampicillin und 0,94 g des mikroporösen Pulvers Accurel[R]
bei einem Preßdruck von 5000 psi eine Tablette hergestellt.
Die Tablette wurde wie in Beispiel I, jedoch unter Einsatz
von Wasser als Lösungsmittel, bei einer Fließgeschwindigkeit
von 100 ml/h geprüft. Abb. 5 ist eine graphische Darstellung
des im Verhältnis zur Zeit freigesetzten Ampicillins in %,
aus der hervorgeht, daß der gesamte Freisetzungsvorgang
wobei als Lösungsmittel Wasser verwendet wurde, bei einer
Fließgeschwindigkeit von 100 ml/h ca. 125 Minuten in Anspruch
nahm. Die Freisetzung verlief in einem Zeitraum von ca.
30 - ca. 120 Minuten, in dem die Freisetzung von ca. 40 bis
98 % der gesamten vorhandenen Ampicillinmenge erfolgte,
relativ konstant.

Beispiel V

Gemäß dem Verfahren von Beispiel I wurde aus 1,0 g Ampicillin
und 0,17 g des mikroporösen Polypropylens Accurel[R] bei
einem Preßdruck von 8000 psi eine Tablette hergestellt. Die
Tablette wurde analog Beispiel I mit einer 5%igen Glucose-

A3AM45128

lösung bei einer Geschwindigkeit von 100 ml/h geprüft.

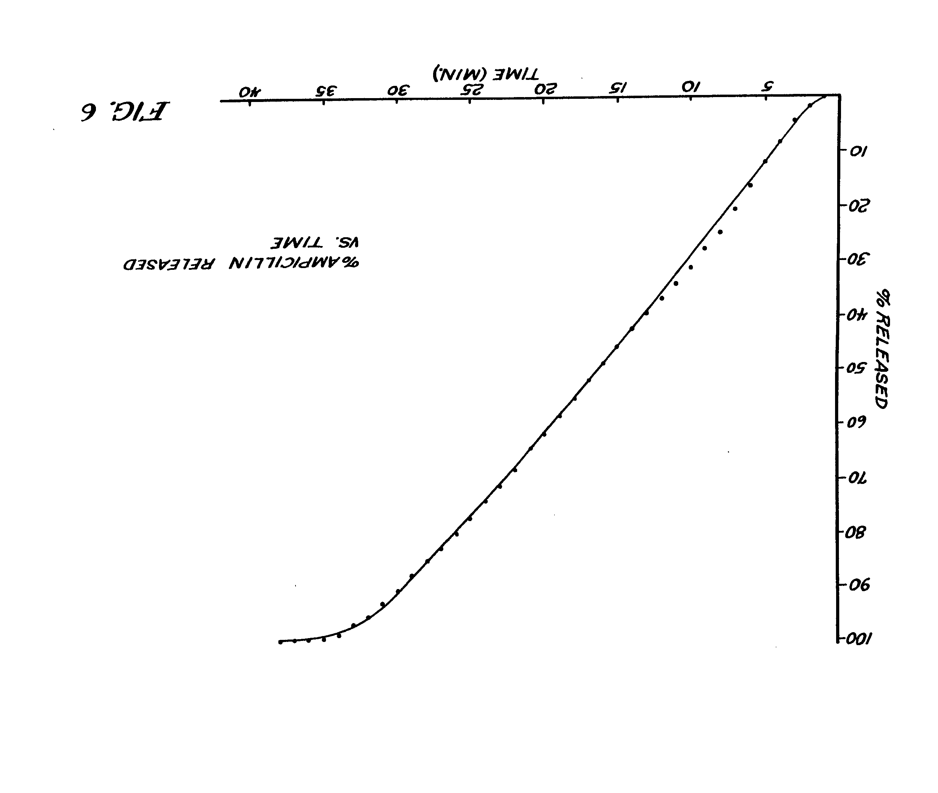

Abb. 6 zeigt eine graphische Darstellung des im Verhältnis
zur Zeit freigesetzten Ampicillins in %, aus der hervorgeht, daß der gesamte Freisetzungsvorgang innerhalb von
ca. 35 Minuten erfolgte, wobei die Freisetzungsgeschwindigkeit während eines Zeitraums von ca. 2 - ca. 32 Minuten,
auf den die Freisetzung von ca. 2 bis ca. 96 % der gesamten
vorhandenen Ampicillinmenge entfiel, nahezu konstant blieb.

Beispiel VI - VIII

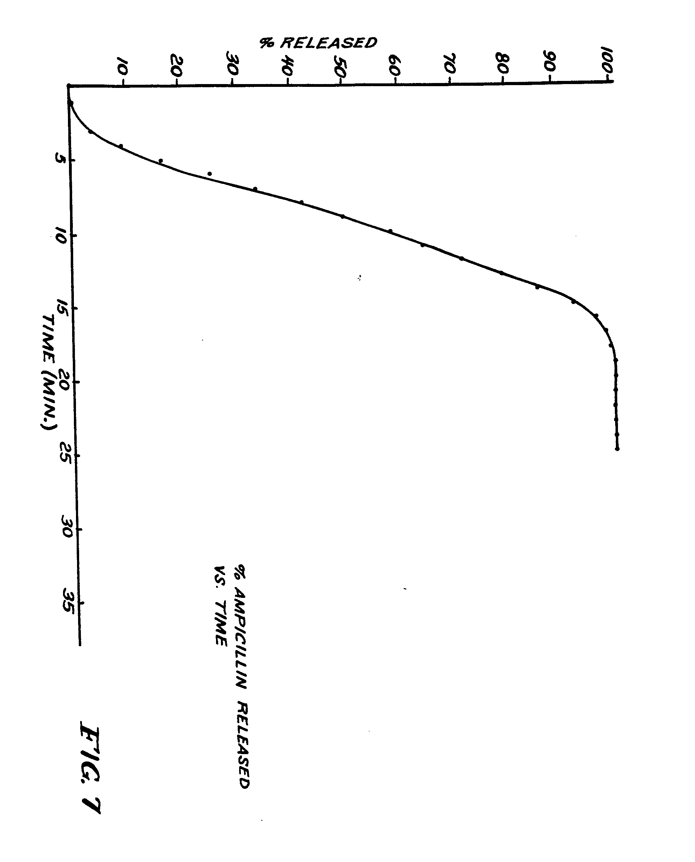

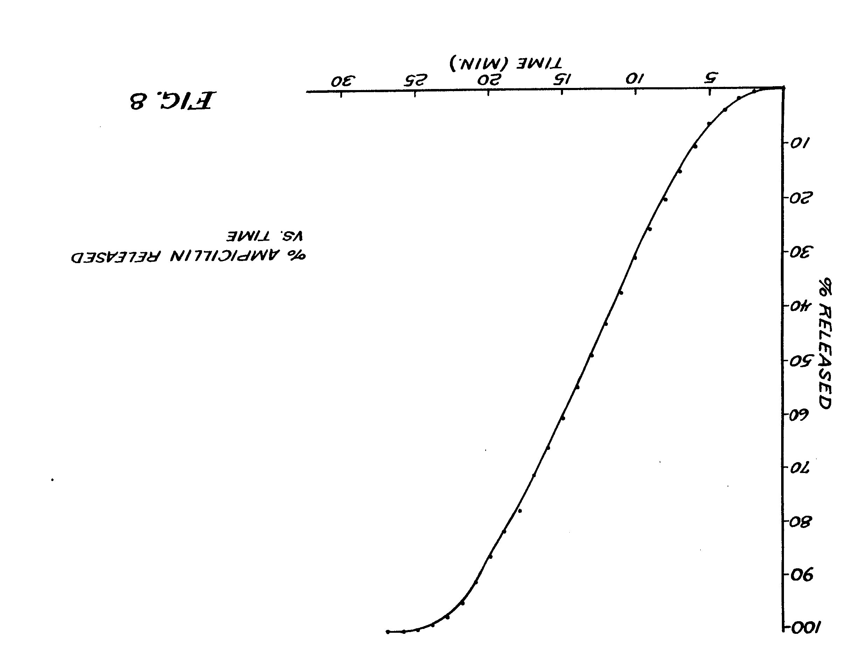

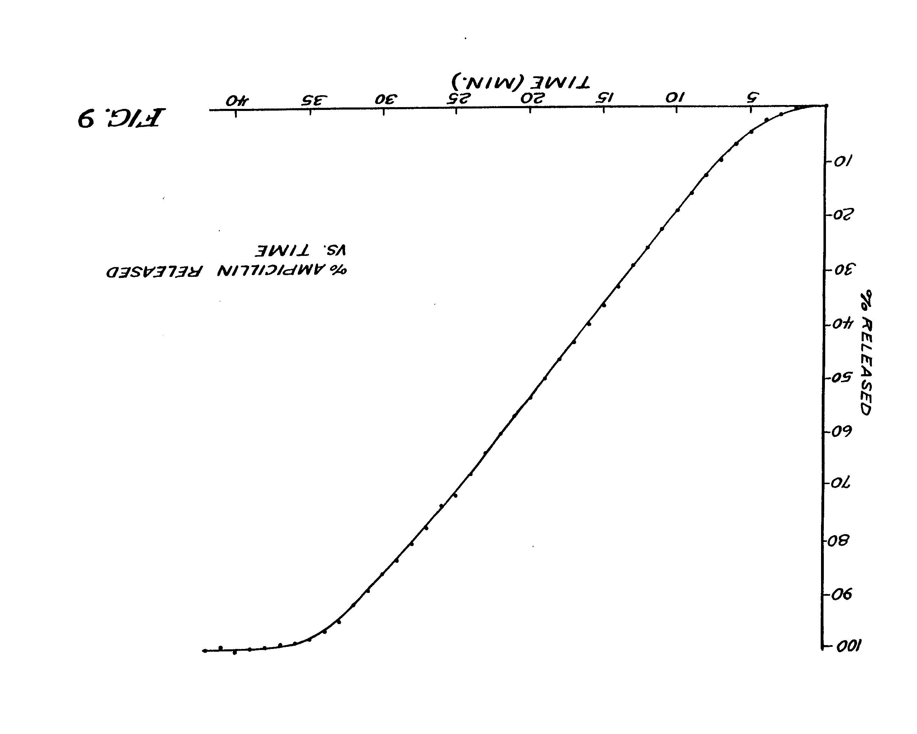

Analog Beispiel I wurden Tabletten mit einem Ampicillin/
Pulver-Verhältnis von 6 : 1 unter Einsatz des mikroporösen
Pulvers Accurel[R] hergestellt. Dabei wurden Tabletten mit
0,4 g Ampicillin (Beispiel VI), 1,0 g Ampicillin (Beispiel
VII) bzw. 2,00 g Ampicillin (Beispiel VIII) hergestellt. Die
Tabletten wurden analog Beispiel I, jedoch unter Einsatz
von Wasser als Lösungsmittel, bei einer Fließgeschwindigkeit
von 100 ml/h geprüft. Die Abbildungen 7, 8 und 9 sind
graphische Darstellungen des im Verhältnis zur Zeit freigesetzten Ampicillins für die Beispiele VI, VII und VIII. Aus
den Abbildungen geht hervor, daß bei allen Tabletten über
einen beträchtlichen Zeitraum nahezu konstante Freisetzungsgeschwindigkeiten erzielt wurden, daß jedoch die absolute
Freisetzungsgeschwindigkeit und die Gesamtdauer der Freisetzung bei den einzelnen Proben unterschiedlich waren.
So erhöhten sich erwartungsgemäß die Zeiten für die Freisetzung der gesamten Ampicillinmenge mit der in den Proben
vorhandenen Ampicillinmenge.

Beispiel IX und X

Gemäß Beispiel I wurden Tabletten aus 0,86 g Ampicillin und 0,14 g des mikroporösen Pulvers Accurel$^{(R)}$ bei Preßdrücken
von 3000 psi (Beispiel IX) und 6000 psi (Beispiel X) hergestellt. Die Tabletten wurden analog Beispiel I, jedoch
unter Einsatz von Wasser als Lösungsmittel, bei einer
Fließgeschwindigkeit von 100 ml/h geprüft. Abb. 10 ist
eine graphische Darstellung des im Verhältnis zur Zeit
freigesetzten Ampicillins in %, aus der hervorgeht, daß
bei beiden Beispielen die gesamte Menge innerhalb von
ca. 23 Minuten freigesetzt wurde und daß die Freisetzungsgeschwindigkeit während eines Zeitraums von ca. 5 und
ca. 20 Minuten, auf den die Freisetzung von ca. 10 bis
90 Prozent der gesamten vorhandenen Ampicillinmenge entfiel, nahezu konstant blieb. Somit scheint das Freisetzungsverhalten der Tablette relativ unabhängig von der angewandten Druckkraft zu sein, sofern zum Formen der Tablette
eine ausreichende Druckkraft angewandt wurde.

## Beispiel XI bis XVII

Um nachzuweisen, daß eine nahezu konstante Freisetzungsgeschwindigkeit auch bei unterschiedlichen Fließgeschwindigkeiten erreicht werden kann, wurden Tabletten aus 0,5 g
Ampicillin und 0.083 g des mikroporösen Pulvers Accurel$^{(R)}$
bei einem Preßdruck von ca. 5350 psi hergestellt. Die
Tabletten wurden in der gleichen Weise wie in Beispiel I
geprüft, wobei jedoch als Lösungsmittel Wasser eingesetzt
und folgende Fließgeschwindigkeiten angewandt wurden.

|  |  |
|---|---|
| Beispiel XI | 2,45 ml/Minute |
| Beispiel XII | 2,18 ml/Minute |
| Beispiel XIII | 1,83 ml/Minute |
| Beispiel XIV | 1,72 ml/Minute |
| Beispiel XV | 1,54 ml/Minute |
| Beispiel XVI | 1,08 ml/Minute |
| Beispiel XVII | 0,89 ml/Minute |

Abb. 11 zeigt für jedes der genannten Beispiele graphische
Darstellungen des im Verhältnis zur Zeit freigesetzten
Ampicillins in %, woraus hervorgeht, daß die absolute
Freisetzungsgeschwindigkeit eine Funktion der Fließgeschwindigkeit ist, daß jedoch während eines beträchtlichen
Teils der Freisetzungskurven für die einzelnen Poren
die Freisetzungsgeschwindigkeit für die einzelnen Fließgeschwindigkeiten relativ konstant bleibt. Wenn sich die
Fließgeschwindigkeit erhöht, erhöht sich auch die absolute
Freisetzungsgeschwindigkeit.

## Beispiele XVIII - XXII

Um nachzuweisen, daß sich die nahezu konstanten Freisetzungsgeschwindigkeiten auch bei unterschiedlichen Tablettendurchmessern erzielen lassen, wurden aus 0,5 g Ampicillin und
0,083 g des mikroporösen Pulvers Accurel[R] mit einem
Preßdruck von ca. 4800 psi Tabletten mit den unten angegebenen Durchmessern hergestellt. Die Tabletten wurden in
der gleichen Weise wie in Beispiel I geprüft, wobei jedoch
als Lösungsmittel Wasser eingesetzt und folgende Fließgeschwindigkeiten angewandt wurden:

|  |  | Tablettendurchmesser |
|---|---|---|
| Beispiel XVIII | 1,61 ml/Minute | 1,25 in. |
| Beispiel XIX | 1,74 ml/Minute | 0,56 in. |
| Beispiel XX | 1,73 ml/Minute | 0,56 in. |
| Beispiel XXI | 1,78 ml/Minute | 0,75 in. |
| Beispiel XXII | 2,25 ml/Minute | 0,56 in. |

Abb. 12 zeigt für die einzelnen Beispiele graphische Darstellungen des im Verhältnis zur Zeit freigesetzten
Ampicillins in %, woraus hervorgeht, daß die absolute Freisetzungsgeschwindigkeit vom Tablettendurchmesser relativ
unabhängig ist und daß die Freisetzungsgeschwindigkeit
für die einzelnen Tablettendurchmesser während eines
beträchtlichen Teils der Freisetzungskurve für die
einzelnen Proben nahezu konstant bleibt.

Beispiele XXIII - XXVI

Um nachzuweisen, daß die hydrophoben/hydrophilen Eigenschaften des inerten Materials sich auf das Freisetzungsverhalten auswirken, wurden aus 0,5 g Ampicillin und verschiedenen Mengen mikrocristalliner Zellulose der Marke
Avicel, wie unten angegeben, bei einer Preßkraft von
ca. 4800 psi Tabletten hergestellt. Die Tabletten wurden
in der gleichen Weise wie in Beispiel I geprüft, wobei
jedoch als Lösungsmittel Wasser eingesetzt wurde und die
Fließgeschwindigkeiten den unten angegebenen Werten entsprachen. Die Tablette des Beispiels XVIII wurde als Vergleichsmuster verwendet.

| Beispiel XXIII | 2,20 ml/Minute | 0,080 g Cellulose |
| Beispiel XXIV | 1,71 ml/Minute | 0,080 g Cellulose |
| Beispiel XXV | 1,53 ml/Minute | 0,050 g Cellulose |
| Beispiel XXVI | 1,69 ml/Minute | 0,075 g Cellulose |
| Beispiel XVIII | 1,51 ml/Minute | 0,083 g Polypropylen |

Abb. 13 zeigt für die einzelnen Beispiele graphische Darstellungen des im Verhältnis zur Zeit freigesetzten
Ampicillins in %, woraus hervorgeht, daß das Freisetzungsverhalten eine Funktion der Menge des zur Herstellung der
Tablette eingesetzten inerten Materials ist, wenn das inerte
Material hydrophil ist. Bei einer Erhöhung der Menge des
hydrophilen inerten Materials entwickelt sich das Freisetzungsverhalten in Richtung einer Kinetik 0. Ordnung.

- 26 -

0168522
A3AM45128

## Beispiele XXVII - XXIX

Um nachzuweisen, daß die hydrophoben/hydrophilen Eigenschaften des inerten Materials sich auf das Freisetzungsverhalten auswirken, wurden aus 0,5 g Ampicillin und verschiedenen Mengen von mikroporösen Polyamid-Pulver bei
einem Preßdruck von ca. 4800 psi Tabletten hergestellt.
Die Tabletten wurden in der gleichen Weise wie in
Beispiel I geprüft, wobei jedoch als Lösungsmittel Wasser
eingesetzt wurde und die Fließgeschwindigkeiten den unten
angegebenen Werten entsprachen. Die Tablette von Beispiel
XVIII wurde als Vergleichsmuster verwendet.

| | | |
|---|---|---|
| Beispiel XXVII | 1,70 ml/Minute | 0,080 g Polyamid |
| Beispiel XXVIII | 1,69 ml/Minute | 0,160 g Polyamid |
| Beispiel XXIX | 1,82 ml/Minute | 0,500 g Polyamid |
| Beispiel XVIII | 1,51 ml/Minute | 0,083 g Polypropylen |

Abb. 14 zeigt für die einzelnen Beispiele graphische Darstellungen des im Verhältnis zur Zeit freigesetzten
Ampicillins in %, woraus hervorgeht, daß das Freisetzungsverhalten eine Funktion der Menge des zur Herstellung der
Tablette verwendeten inerten Materials ist, wenn das inerte
Material hydrophil ist. Bei einer Erhöhung der Menge des
hydrophilen inerten Materials entwickelt sich das Freisetzungsverhalten in Richtung einer Kinetik 0. Ordnung.

## Beispiel XXX

Um nachzuweisen, daß das Freisetzungsverhalten sich nach der
Löslichkeit des Wirkstoffes im Lösungsmittel richtet, wurde
aus 0,5 g Acetaminophen und 0,083 g des mikroporösen Pulvers
Accurel[R] bei einer Druckkraft von ca. 4800 psi eine Tablette hergestellt. Die Tablette wurde in der gleichen
Weise wie in Beispiel I geprüft, wobei jedoch als
Lösungsmittel Wasser verwendet wurde und die Fließgeschwindigkeit 1,80 ml/Minute betrug.

Die Tablette von Beispiel XVIII wurde zu einem Vergleich
bei einer Fließgeschwindigkeit von 1,61 ml/Minute verwendet.

Abb. 15 zeigt für die einzelnen Beispiele graphische Darstellungen des im Verhältnis zur Zeit freigesetzten Wirkstoffs in %, woraus hervorgeht, daß sich das Freisetzungsverhalten nach der Löslichkeit des Wirkstoffs im Lösungsmittel richtet, da Ampicillin (Beispiel XVIII) eine Wasserlöslichkeit von ca. 13,6 % und Acetaminophen eine Wasserlöslichkeit nur von 1,4 % besitzen und unter den Bedingungen
von Beispiel XXX nur eine Freisetzungskinetik gemäß der
klassischen Quadratwurzel-der-Zeit-Kinetik aufwiesen.

Beispiel XXXI

Um nachzuweisen, daß die nahezu konstante Freisetzungsgeschwindigkeit sich mit einem Wirkstoff mit einer Wasserlöslichkeit von nur ca. 7 % erreichen läßt, wurden aus 0,5 g
Hydrochinon und 0,083 g des mikroporösen Pulvers Accurel[(R)]
mit einer Preßkraft von ca. 4800 psi Tabletten hergestellt.
Die Tablette wurde in der gleichen Weise wie in Beispiel I
geprüft, wobei jedoch als Lösungsmittel Wasser verwendet
wurde und die Fließgeschwindigkeit ca. 1,62 ml/Minute betrug.
Die Ampicillin mit einer Löslichkeit von ca. 13,6 % enthaltende Tablette von Beispiel XVIII wurde für Vergleichszwecke bei einer Fließgeschwindigkeit von 1,51 ml/Minute
eingesetzt.

Abb. 16 zeigt für die einzelnen Beispiele graphische Darstellungen des im Verhältnis zur Zeit freigesetzten Wirkstoffs in %, woraus hervorgeht, daß sich das Freisetzungsverhalten nach der Löslichkeit des Wirkstoffes richtet, daß jedoch die Freisetzungsgeschwindigkeit der einzelnen Wirkstoffe in beträchtlichen Teilen der Freisetzungskurven für die einzelnen Proben relativ konstant bleibt. Mit zunehmender Löslichkeit erhöht sich auch die absolute Freisetzungsgeschwindigkeit.

0168522

A3AM45128

Patentansprüche

1. Formkörper mit einheitlicher Struktur aus ca. 10 bis 90 Gew.-% eines Wirkstoffes und ca. 90 bis 10 Gew.-% eines synthetischen thermoplastischen Polymermaterials, dadurch gekennzeichnet, daß der Formkörper einen im wesentlichen physikalischen Zusammenhalt aufweist und daß der Wirkstoff gleichmäßig in einer mit der Außenfläche des Formkörpers kommunizierenden Weise im gesamten Formkörper verteilt ist, wobei der Wirkstoff zwar eine Löslichkeit in Wasser aufweist, er aber auf Grund der Bildung von Agglomeraten oder Gelteilchen in Wasser schwer löslich oder schwer verteilbar ist und/ oder auf Grund seiner chemischen Instabilität in wäßriger Lösung nur kurze Zeit beständig ist.

2. Formkörper nach Anspruch 1, dadurch gekennzeichnet, daß das Polymermaterial hydrophob ist.

3. Formkörper nach einem der Ansprüche 1 oder 2, dadurch gekennzeichnet, daß der Wirkstoff Ampicillin ist.

4. Formkörper nach einem der Ansprüche 1 oder 2, dadurch gekennzeichnet, daß der Wirkstoff ein adrenokortikotropisches Hormon (ACTH), Bleomycin, Cytoxinarbinosid, Diphenylhydramin, ein gonadotropinfreisetzendes Hormon (GnRH), Insulin, Parathormon, Angiotensin II, Chlordiacepoxyd, Diäthylcarbamizin, Dopamin, Heparin, Nitrofurantoin oder Pilocarpin ist.

0168522

A3AM45128

5. Formkörper nach einem der Ansprüche 1 oder 2, dadurch gekennzeichnet, daß der Wirkstoff Mithramycin, Natrium-Warfarin, Pentothal, Daunorubicin-Hydrochlorid, menschliches Choriongonadotropin, Amoxicillin, Natrium-Cefazolin, Cephaloridin, Mitomycin, Cyclophosphamid, Asparaginase, Natrium-Methicillin, Polymyxin-B-Sulfat, Tetracyclin-Hydrochlorid, Minocyclin-Hydrochlorid, Kalium-Penicillin V oder Natrium-Cloxacillin ist.

6. Formkörper nach einem der Ansprüche 1 oder 2, dadurch gekennzeichnet, daß der Wirkstoff in Wasser eine Löslichkeit von mindestens 5 % aufweist.

7. Verfahren zur Herstellung eines Formkörpers nach einem der Ansprüche 1 bis 6, dadurch gekennzeichnet, daß man 10 bis 90 Gew.-% Teilchen des Wirkstoffs mit 10 bis 90 Gew.-% Polymerteilchen, wobei die Polymerteilchen einen Durchmesser von ca. 40 bis ca. 400 µm aufweisen und zu ca. 0 bis ca. 100 Gew.-% aus mikroporösen Teilchen eines synthetischen thermoplastischen Polymermaterials und zu ca. 0 bis ca. 100 Gew.-% aus einem nichtporösen synthetischen thermoplastischen Polymermaterial bestehen, trocken miteinander vermischt, sodann in die gewünschte Form bringt und einem effektiven Druck von ca. 200 bis 8000 psi aussetzt, wobei die Mischung in der gewünschten Form für einen Zeitraum gehalten wird, der ausreicht, um der Mischung einen im wesentlichen physikalischen Zusammenhalt zu verleihen.

8. Verfahren nach Anspruch 1, dadurch gekennzeichnet, daß man ein hydrophobes Polymermaterial verwendet.

9. Verfahren nach einem der Ansprüche 7 oder 8, dadurch gekennzeichnet, daß man einen Wirkstoff verwendet, der in Wasser eine Löslichkeit von mindestens 5 % aufweist.

10. Verfahren nach einem der Ansprüche 7 bis 9, dadurch gekennzeichnet, daß man das Gemisch zu Tabletten formt.

11. Verwendung der Formkörper nach einem der Ansprüche 1 bis 10 zur Freisetzung einer im wesentlichen gleichmäßigen Wirkstoffmenge pro Zeiteinheit, dadurch gekennzeichnet, daß man ein Lösungsmittel für den Wirkstoff mit gesteuerter Geschwindigkeit in ein mit einer Einlaß- und einer Abflußöffnung, die in einem gewissen Abstand voneinander angebracht sind, versehenes Gefäß einleitet, wobei das Gefäß so angeordnet ist, daß es darin zu keiner wesentlichen Ansammlung von konzentrierter Wirkstofflösung kommt, und wobei das Gefäß den Formkörper enthält und man das Lösungsmittel, das den darin gelösten oder verteilten Wirkstoff enthält, aus dem Gefäß durch die Abflußöffnung mit einer gesteuerten Geschwindigkeit ableitet.

12. Verwendung nach Anspruch 11, dadurch gekennzeichnet, daß das Gefäß so angeordnet ist, daß die Ableitung der Flüssigkeit aus dem Gefäß in Folge der Schwerkraft erfolgt.

13. Verwendung nach einem der Ansprüche 11 oder 12, dadurch gekennzeichnet, daß man einen tablettenförmigen Formkörper aus dem Polymermaterial Polyamid und dem Wirkstoff Ampicillin verwendet.

0168522

A3AM45128

14. Verwendung nach Anspruch 13, dadurch gekennzeichnet, daß man einen Formkörper aus gleichen Teilen Polyamid und Ampicillin verwendet.

**FIG. 1**

% AMPICILLIN RELEASED
VS. TIME

FIG. 2

% AMPICILLIN RELEASED
VS. TIME

FIG. 3

4/16

% RELEASED

1.0

0.5

8    16    24    32    40    48    56    64    72    80    82

TIME (MIN.)

% AMPICILLIN RELEASED
VS TIME

FIG. 4

% AMPICILLIN RELEASED
VS. TIME

TIME (MIN.)

FIG. 5

0168522

6/6

% AMPICILLIN RELEASED
VS. TIME

FIG. 6

% AMPICILLIN RELEASED
VS. TIME

FIG. 7

8/16

% AMPICILLIN RELEASED
VS. TIME

FIG. 8

% AMPICILLIN RELEASED VS. TIME

FIG. 9

0168522

10/16

% AMPICILLIN RELEASED
VS. TIME

o  EXAMPLE IX
•  EXAMPLE X

FIG. 10

% RELEASED

TIME (MIN.)

% AMPICILLIN RELEASED vs. TIME

- • EXAMPLE XI
- ✳ EXAMPLE XII
- ◁ EXAMPLE XIII
- ▷ EXAMPLE XIV
- ○ EXAMPLE XV
- + EXAMPLE XVI
- × EXAMPLE XVII

FIG.11

FIG. 12

% AMPICILLIN RELEASED
VS. TIME

X    EXAMPLE XXII
+    EXAMPLE XXI
o    EXAMPLE XX
·    EXAMPLE XIX
⊙    EXAMPLE XVIII

TIME (MIN.)

% RELEASED

12/16          0168522

FIG. 13

% AMPICILLIN RELEASED
VS. TIME

× EXAMPLE XXIII
+ EXAMPLE XXIV
△ EXAMPLE XXV
• EXAMPLE XXVI
● EXAMPLE XVIII

FIG. 14

% AMPICILLIN RELEASED VS. TIME

⊙ EXAMPLE XVIII
+ EXAMPLE XXVII
• EXAMPLE XXVIII
× EXAMPLE XXIX

15/16

0168522

% RELEASED

% AMPICILLIN RELEASED
VS. TIME

⊙ EXAMPLE XVIII
• EXAMPLE XXX

FIG. 15

TIME (MIN.)

0168522

% AMPICILLIN RELEASED VS. TIME

⊙ EXAMPLE XVIII
• EXAMPLE XXXI

FIG.16